# EUROPEAN PATENT APPLICATION

(11) **EP 4 650 355 A1**
(43) Date of publication of application: **19.11.2025**
(21) Application number: 24741192.9
(22) Date of filing: 08.01.2024
(51) Int. Cl.: C07D 471/16, C07D 491/16

(54) **PREPARATION METHOD FOR NITROGEN-CONTAINING FUSED RING COMPOUND**

(30) Priority: 09.01.2023 CN 202310025425
(71) Applicant: Sichuan Kelun-Biotech Biopharmaceutical Co., Ltd., Chengdu, Sichuan 611138 (CN)
(72) Inventor: LIANG, Yong, Chengdu, Sichuan 611138 (CN); TANG, Wanlong, Chengdu, Sichuan 611138 (CN); XIA, Dongliang, Chengdu, Sichuan 611138 (CN); CHEN, Kai, Chengdu, Sichuan 611138 (CN); QI, Hongxia, Chengdu, Sichuan 611138 (CN); LIANG, Yufeng, Chengdu, Sichuan 611138 (CN); SONG, Hongmei, Chengdu, Sichuan 611138 (CN); GE, Junyou, Chengdu, Sichuan 611138 (CN)
(74) Representative: Meissner Bolte Partnerschaft mbB
(86) International application number: PCT/CN2024/071117
(87) International publication number: WO 2024/149193

(57) **Abstract**

The present application provides a preparation method for a nitrogen-containing fused ring compound having anti-tumor activity. According to the method, a target product can be efficiently obtained under relatively mild reaction conditions, and upon comparison with the original processes, the yield rate is remarkably improved, and the material cost is greatly reduced, thereby better facilitating process scale-up.

## Description

The present application is based on CN application No. 202310025425.7, filed on January 9, 2023, and claims its priority. The disclosure of the CN application is incorporated herein by reference in its entirety.

### Technical Field

The present application relates to the field of pharmaceutical chemical industry, and specifically to a preparation method for a nitrogen-containing fused ring compound.

### Background Art

Camptothecin (CPT) is a pentacyclic quinoline-based parent compound isolated from the plant *Camptotheca acuminata Decne* of the *Nyssaceae* family. It consists of quinoline ring AB, pyrrole ring C, pyridone ring D and α-hydroxy-lactone ring E, with the 20-position in *S* configuration. It was introduced into clinical practice in the early 1970s due to its excellent anticancer activity. Later, the clinical trial was terminated because of severe side effects such as diarrhea and hemorrhagic cystitis.

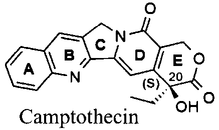

Research data reveal that camptothecin can form a three-membered ring complex with cellular DNA topoisomerase I, consequently inhibiting DNA unwinding, resulting in DNA replication blockage and cell death (Cancer Res. 1989, 49, 6365). Camptothecin and its derivatives exhibit potent anti-tumor activity in animal models of cancers such as lung cancer, breast cancer, colorectal cancer, and ovarian cancer (Nature Review Cancer. 2006, 6, 789).

Currently, several camptothecin drugs have been approved for marketing for tumor treatment. For instance, topotecan is used for the treatment of ovarian cancer, belotecan is used for the treatment of ovarian cancer and small cell lung cancer. Dxd, developed by Daiichi Sankyo, was granted approval by the FDA on August 11, 2022, becoming the world's first HER2-targeted antibody-drug conjugate Trastuzumab deruxtecan (T-DXd, DS-8201). At present, this drug has achieved remarkable "results" in common solid tumors such as non-small cell lung cancer, breast cancer, gastric cancer, and colorectal cancer.

The synthesis process of camptothecin analogs holds significant implications for the commercialization of anti-tumor drugs. For example, the control of impurities and the large-scale production of camptothecin analogs will substantially impact the production cost of anti-tumor drugs.

Daiichi Sankyo disclosed a synthesis route for camptothecin analogs in patent application CN111065621A. However, numerous aspects of the synthesis method require improved. For instance, the two-step high-temperature reactions have high equipment requirements, long reaction time, and low yield of target product (total yield of two steps is 43%). Isomers are difficult to recycle, isomer control becomes challenging after process scale-up, and the synthesis efficiency is low. These ultimately lead to high cost of scale-up production.

### Contents of the present invention

In order to solve the above technical problems, the present application provides a preparation method for obtaining exatecan in singular configuration and analogs thereof as shown in Formula (I), wherein R₁ and R₂ in Formula (I) are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₄ haloalkyl, hydroxyl and cyano; or R₁ and R₂, together with the carbon atoms to which they are connected, form a 5- to 6-membered carbon ring or an oxygen-containing heterocyclic ring. In some embodiments, R₁ and R₂ are independently selected from halogen and C₁₋₆ alkyl. In some embodiments, R₁ and R₂ are independently selected from the group consisting of F, Cl, Br, I and C₁₋₂ alkyl. In some embodiments, R₁ and R₂ are independently selected from Cl and methyl. In some embodiments, R₁ is Cl and R₂ is methyl.

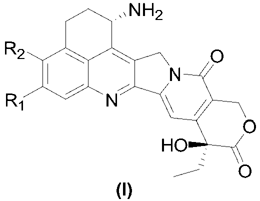

The embodiments comprise obtaining a parent core structure as shown in Formula (III) under mild conditions, then inverting the isomer into a target configuration as shown in Formula (IV) under acidic conditions, followed by deprotecting and salifying, to obtain the target compound as shown in Formula (I) in singular configuration, through three steps with an overall yield of >75%. The method is highly versatile and can be further applied to the synthesis of other exatecan analogs. It has a simple process, facilitates easy control of isomers, ensures high synthesis efficiency, incurs low cost, and is more conducive to process amplification and more practical.

Specifically, the present application provides a synthesis method suitable for large-scale preparation of exatecan in singular configuration and analogs thereof, and the synthesis route is as follows: 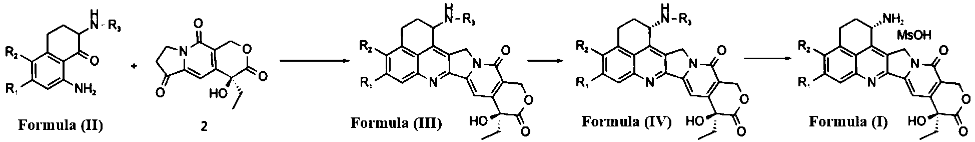 wherein R₁ and R₂ are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₄ haloalkyl, hydroxyl and cyano; or R₁ and R₂, together with the carbon atoms to which they are connected, form a 5-to 6-membered carbon ring or an oxygen-containing heterocyclic ring; preferably, R₁ and R₂ are independently selected from halogen and C₁₋₆ alkyl; preferably, R₁ and R₂ are independently selected from the group consisting of F, Cl, Br, I and C₁₋₂ alkyl; preferably, R₁ and R₂ are independently selected from Cl and methyl; preferably, R₁ is Cl and R₂ is methyl;
wherein R₃ is an electron-withdrawing protective group, for example, a substituted ethoxycarbonyl (e.g., 2,2,2-trichloroethoxycarbonyl, 2-trimethylsilylethoxycarbonamide), formyl, acetyl, chloroacetyl, dichloroacetyl, trichloroacetyl, trifluoroacetyl, Fmoc, Fmoc-like group, Troc, Cbz, Teoc, Alloc, phthaloyl (Pht), trifluoromethanesulfonyl, tert-butylsulfonyl, methylsulfonyl, benzenesulfonyl, p-toluenesulfonyl, benzylsulfonyl, 2-(trimethylsilyl)ethanesulfonyl, 4-nitrobenzenesulfonyl, (9H-9-pentyl)methylsulfonyl, 2- or 4-nitrobenzenesulfonyl, 2,4-dinitrobenzenesulfonyl, pivaloyl.

In one aspect, the present application provides a method for preparing a compound as shown in Formula (III), which comprises a step of reacting a compound as shown in Formula (II) and Compound 2 in the presence of a catalyst; 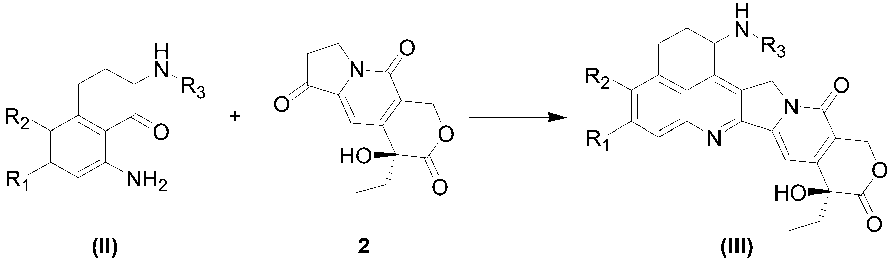 wherein, R₁ and R₂ are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₄ haloalkyl, hydroxyl and cyano; or R₁ and R₂, together with the carbon atoms to which they are connected, form a 5-to 6-membered carbon ring or an oxygen-containing heterocyclic ring; preferably, R₁ and R₂ are independently selected from halogen and C₁₋₆ alkyl; preferably, R₁ and R₂ are independently selected from the group consisting of F, Cl, Br, I and C₁₋₂ alkyl; preferably, R₁ and R₂ are independently selected from Cl and methyl; preferably, R₁ is Cl and R₂ is methyl;
R₃ is an electron-withdrawing protective group;
the catalyst is selected from one or more of PPTS, AcOH, TFA, H₂SO₄, proline, PPA, P₂O₅, CAN, T₃P, KOH, I₂, MgCl₂ and TMSCl.

In some embodiments, the catalyst is PPA.

In some embodiments, the compound as shown in Formula (II) and Compound 2 are reacted in a solvent, wherein the solvent is selected from the group consisting of aromatic hydrocarbons (e.g., toluene, xylene, o-toluene, m-toluene), aliphatic hydrocarbons (e.g., hexane, n-heptane), alcohols (e.g., methanol, ethanol, isopropanol), organic acids (e.g., acetic acid, trifluoroacetic acid), phenols (e.g., phenol, o-cresol, m-cresol, p-cresol), ethers (e.g., ethyl ether, ethylene oxide, anisole), esters (e.g., methyl acetate, ethyl acetate, propyl acetate), ketones (e.g., acetone, butanone), amides (e.g., DMF, DMA), nitriles (e.g., acetonitrile), heterocyclics (e.g., NMP, 1,4-dioxane, 2-MeTHF), sulfur-containing organic solvents (e.g., DMSO), and any combination thereof.

In some embodiments, the solvent used in the method is a combination of a phenolic solvent and a heterocyclic solvent.

In some embodiments, the organic solvent is a mixed solvent of o-cresol and 1,4-dioxane.

More specifically, in one aspect, the present application provides a method for preparing a compound as shown in Formula (III), comprising a step of reacting a compound as shown in Formula (II) with Compound **2** in the presence of PPA in a mixed solvent of o-cresol and 1,4-dioxane; 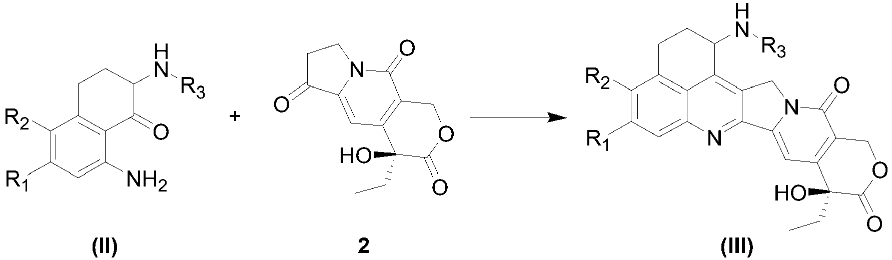 wherein, R1, R₂ and R₃ are defined as described above.

Studies indicate that the reaction reagents greatly influence the reaction rate, product selectivity, product stability, and impurities. In some embodiments, an acidic catalyst such as p-toluenesulfonic acid, PPTS, AcOH, TFA, H₂SO₄, proline, phosphoric acid, or other catalysts such as P₂O₅, CAN, T₃P, KOH, I₂, MgCl₂ or TMSCl can be selected to replace PPA, but PPA is more preferred.

At the same time, it has also been found in studies that the reaction solvent also greatly influence the reaction rate and reaction by-products. In some embodiments, the reaction solvent is PhMe, xylene, AcOH, TFA, phenol, o-toluene, m-toluene, p-cresol, EtOH, DMF, DMSO, DMA, NMP, anisole, ACN, butanone, n-heptane, ethyl acetate, 1,4-dioxane and 2-MeTHF, and a mixed solvent system of these solvents. However, a mixed solvent system of o-cresol and 1,4-dioxane is more preferred.

In some embodiments, the volume ratio of o-cresol:1,4-dioxane is (10:1) to (1:10), such as (10:1) to (1:1), (9:1) to (1:1), (8:1) to (1:1), (7:1) to (1:1), (6:1) to (1:1), (5:1) to (1:1), (4:1) to (1:1), (3:1) to (1:1), (2:1) to (1:1), preferably 1:1.

In some embodiments, the feed molar ratio of the compound as shown in Formula (II) to Compound 2 is (1:1) to (1:1.5). For instance, it may be (0.8:1) to (1:1.5). As further examples, it may be (1:1) to (1:1.1), (1:1) to (1:1.2), (1:1) to (1:1.3), or (1:1) to (1:1.4), preferably 1:1.2.

In some embodiments, based on the compound as shown in Formula (II), the feed equivalent of PPA is 0.2 to 5 equivalents, such as 0.2 equivalents, 0.3 equivalents, 0.4 equivalents, 0.5 equivalents, 0.6 equivalents, 0.7 equivalents, 0.8 equivalents, 0.9 equivalents, 1.0 equivalents, 1.1 equivalents, 1.2 equivalents, 1.3 equivalents, 1.4 equivalents, 1.5 equivalents, 1.6 equivalents, 1.7 equivalents, 1.8 equivalents, 1.9 equivalents, 2.0 equivalents, 2.5 equivalents, 3.0 equivalents, 3.5 equivalents, 4.0 equivalents, 4.5 equivalents or 5.0 equivalents, preferably 1.0 equivalents.

In some embodiments, the compound as shown in Formula (II) and Compound 2 are reacted at 60 °C to 140 °C, for example, 60 °C, 65 °C, 70 °C, 75 °C, 80 °C, 85 °C, 90 °C, 95 °C, 100°C, 105 °C, 110 °C, 115 °C, 120 °C, 125 °C, 130 °C, 135 °C or 140 °C, preferably 85 °C to 95 °C.

In some embodiments, the compound as shown in Formula (II) and Compound 2 are reacted for 6 to 48 hours, for example, 6 to 24 hours, 6 to 18 hours, preferably 12 to 18 hours.

In some embodiments, the reaction is carried out without gas (e.g., nitrogen) protection, or with nitrogen protection and argon protection, preferably under nitrogen environment.

In some embodiments, after the reaction is completed, a step of post-treatment to obtain a crude product of the compound as shown in Formula (III) is also comprised.

In some embodiments, the post-treatment step comprises adding water, sodium carbonate aqueous solution or sodium bicarbonate aqueous solution for quenching, and then filtering; or adding a mixed solvent of DCM and DCM-IPA or 2-MeTHF for extraction; or directly concentrating the reaction solution under reduced pressure to remove 1,4-dioxane, and then adding one or more solvents selected from isopropanol, ethyl acetate, acetone, MTBE, PhMe, n-heptane and ACN for dilution, and then filtering,; preferably, concentrating under reduced pressure to remove 1,4-dioxane, and then adding MTBE and n-heptane simultaneously or separately, and then filtering to obtain a crude product of the compound as shown in Formula (III).

In some embodiments, the crude product of the compound as shown in Formula (III) does not need to be dried, preferably, residual solvent is removed under vacuum or blowing conditions at 45 to 55 °C before the next treatment.

In some embodiments, after obtaining the crude product of the compound as shown in Formula (III), a step of washing the crude product of the compound as shown in Formula (III) is also comprised.

In some embodiments, the washing step comprises dissolving the crude product of the compound as shown in Formula (III) with an organic solvent to obtain an organic phase, washing the organic phase with an aqueous phase for 1 to 5 times, and concentrating the organic phase. In some embodiments, the organic solvent is selected from the group consisting of DCM, a mixed solvent system of DCM-IPA, DCM-EtOH, or DCM-MeOH, and 2-MeTHF, preferably 2-MeTHF.

In some embodiments, each aqueous phase is independently selected from the group consisting of water, NaCl aqueous solution, ammonium chloride aqueous solution, sodium carbonate aqueous solution, sodium bicarbonate aqueous solution, sodium hydroxide aqueous solution, disodium hydrogen phosphate aqueous solution, sodium sulfite aqueous solution, sodium thiosulfate aqueous solution, and any combination thereof.

In some embodiments, the organic phase is washed with a NaCl aqueous solution, a mixed solution of sodium sulfite aqueous solution (e.g., 2% sodium sulfite aqueous solution) and saturated NaCl aqueous solution (e.g., saturated NaCl aqueous solution) (e.g., the volume ratio of the sodium sulfate aqueous solution to the NaCl aqueous solution is 1:1), a mixed solution of sodium carbonate aqueous solution (e.g., 2% sodium carbonate aqueous solution) and NaCl aqueous solution (e.g., saturated NaCl aqueous solution) (e.g., the volume ratio of the sodium carbonate aqueous solution to the NaCl aqueous solution is 1:1), and a NaCl aqueous solution, in sequence.

In some embodiments, after washing the crude product of the compound as shown in Formula (III), a recrystallization step is also comprised.

In some embodiments, the recrystallization step comprises dissolving the compound as shown in Formula (III) in 2-MeTHF and precipitating crystals with MTBE. In some specific embodiments, the compound as shown in Formula (III) is added to 2-MeTHF, heated to 40 to 60 °C to obtain a supersaturated solution of the compound as shown in Formula (III), MTBE at 2 to 5 times the volume of 2-MeTHF is added, the temperature is lowered to 20 to 30 °C, and the compound as shown in Formula (III) is collected by filtration. Optionally, after the filtration, a step of washing with MTBE and drying is also comprised.

In the above preparation method, by screening the types and equivalents of reaction reagents and solvents, optimizing the reaction temperature, feed ratio, and post-treatment and refining process, the reaction temperature is lowered, the reaction time is shortened, the reaction stability and yield are enhanced, the post-reaction treatment and refining process are simplified, and the stability and scalability of the process are improved.

In one aspect, the present application provides a method for preparing a compound as shown in Formula (IV), which comprises the following step of converting the compound as shown in Formula (III) into the compound as shown in Formula (IV); 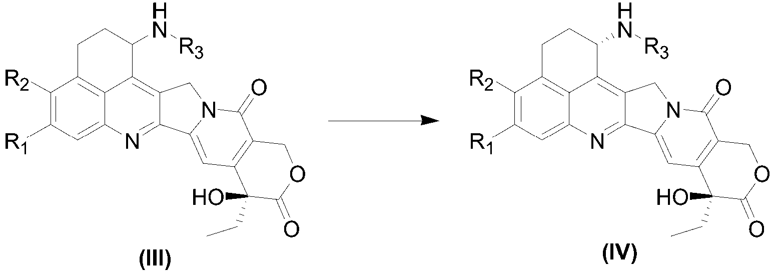 wherein, R₁, R₂ and R₃ are as described above.

In some embodiments, the conversion is carried out in an organic solvent selected from the group consisting of alcohols (e.g., methanol, ethanol, isopropanol, n-butanol), ketones (e.g., acetone, butanone, methyl isobutyl ketone), esters (e.g., ethyl acetate, isopropyl acetate), ethers (e.g., MTBE, isopropyl ether, anisole), aliphatic hydrocarbons (e.g., n-heptane), heterocyclics (e.g., THF, 1,4-dioxane, 2-MeTHF, NMP), amides (e.g., DMF, DMA, sulfur-containing organic solvents (e.g., DMSO), halogenated aliphatic hydrocarbons (e.g., DCM), ammoniums (e.g., CAN), aromatic hydrocarbons (e.g., toluene), and any combination thereof.

In some embodiments, the conversion is carried out in an organic solvent selected from the group consisting of methanol, ethanol, isopropanol, n-butanol, acetone, butanone, methyl isobutyl ketone, ethyl acetate, isopropyl acetate, MTBE, n-heptane, THF, 1,4-dioxane, 2-MeTHF, isopropyl ether, DMF, DMA, DMSO, NMP, anisole, DCM, CAN, toluene and any combination thereof.

In some embodiments, the conversion is carried out in the presence of an acid. In some embodiments, the acid is HCl.

In some embodiments, the conversion is carried out in HCl/1,4-dioxane.

In some embodiments, the acid feed is 2 to 20 equivalents based on the compound as shown in Formula (III).

In some embodiments, the present application provides a method for preparing a compound as shown in Formula (IV), comprising the step of converting the compound as shown in Formula (III) into a compound as shown in Formula (IV) in the presence of HCl; 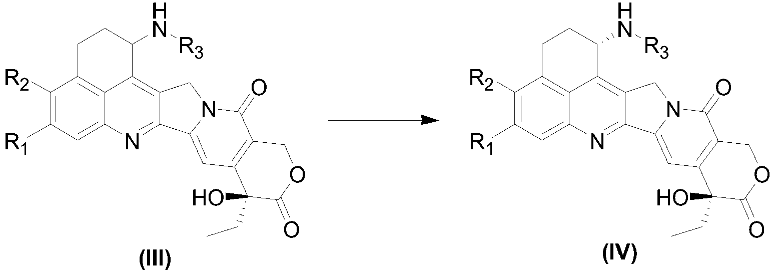 wherein, R1, R₂ and R₃ are as described above.

It has been found in studies that different acids have a significant impact on the stability of raw materials and products, as well as the isomer ratio of the products. In some embodiments, an acid reagent such as concentrated hydrochloric acid, dilute hydrochloric acid, HCl in an organic solvent solution, hydrobromic acid, acetic acid, methanesulfonic acid, p-toluenesulfonic acid, benzoic acid, boron trifluoride in ethyl ether solution, TFA, H₂SO₄ and H₃PO₄, preferably HCl/1,4-dioxane solution, can be added for the conversion.

In some embodiments, the feed amount of HCl/1,4-dioxane is 2 to 20 equivalents, preferably 4 to 8 equivalents.

In some embodiments, the reaction solvent is methanol, ethanol, isopropanol, n-butanol, acetone, butanone, methyl isobutyl ketone, ethyl acetate, isopropyl acetate, MTBE, n-heptane, THF, 1,4-dioxane, 2-MeTHF, isopropyl ether, DMF, DMA, DMSO, NMP, anisole, DCM, ACN and toluene, preferably 1,4-dioxane.

In some embodiments, the conversion is carried out at 25 to 100 °C, preferably 40 to 50 °C, such as 45 °C.

In some embodiments, the conversion is carried out for 1 to 24 hours, such as 4 to 24 hours, 1 to 20 hours, preferably 6 to 10 hours.

In some embodiments, the reaction is stopped when the ratio of the target product to its isomer in the reaction solution is (2:1) to (30:1), for example, (2:1) to (25:1), (2:1) to (20:1), (2:1) to (15:1), (5:1) to (30:1), (5:1) to (25:1), (5:1) to (20:1), (5:1) to (15:1), (10:1) to (30:1), (10:1) to (25:1), (10:1) to (20:1), (10:1) to (15:1), (15:1) to (30:1), (15:1) to (25:1), (15:1) to (20:1), preferably (10:1) to (25:1).

In some embodiments, after the conversion, a post-treatment step is also comprised.

In some embodiments, the post-treatment involves directly concentrating under reduced pressure, or adding one or more anti-solvents of the reaction solvent, such as one or more of ethanol, isopropanol, n-butanol, acetone, butanone, methyl isobutyl ketone, ethyl acetate, isopropyl acetate, MTBE, n-heptane, THF, 1,4-dioxane, isopropyl ether, anisole, DCM, ACN and toluene, precipitating more solids and then filtering, preferably adding MTBE and then filtering to obtain the target product.

In some embodiments, the method further comprises a step of preparing the compound as shown in Formula (III) according to any of the methods described in the first aspect.

After the reaction and treatment under the above specified reaction conditions, the ratio of the compound as shown in Formula (IV) to its isomers increases from about 1:1 to 30~40:1, the purity of the product is further improved, the removal of subsequent isomer impurities becomes easier, the production cost is reduced, and the process is more amendable to scale-up production.

In another aspect, the present application provides a method for preparing a compound as shown in Formula (I) or a salt thereof, which comprises a step of removing the amino protective group of the compound as shown in Formula (IV) to obtain the compound as shown in Formula (I); 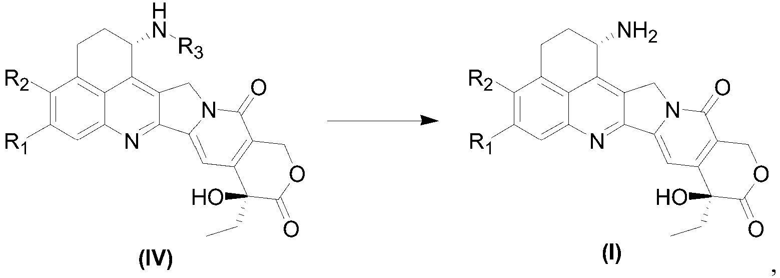 wherein, R1, R₂ and R₃ are as defined above.

The amino protective group can be removed by a method known in the art. For example, when R₃ is Fmoc, Et₂NH can be added to carry out the deprotection reaction.

In some embodiments, after removing the amino protective group of the compound as shown in Formula (IV), a post-treatment step is also comprised.

In some embodiments, the post-treatment step involves concentrating, slurry washing and filtering for 1 to 3 times to obtain a crude product of the compound as shown in Formula (I).

In some embodiments, after concentrating to 1/4 to 1/2, preferably 1/3 of the volume of the original solution, acetonitrile is added for slurrying, and the concentration is continued to be complete.

In some embodiments, the slurry washing is carried out at a high temperature of 60 to 80 °C (preferably 65 to 75 °C) and a low temperature of 10 to 40 °C (preferably 20 to 30 °C) in sequence, and then the solid is collected by filtration to obtain a crude product of the compound as shown in Formula (IV).

In some embodiments, after completing the post-treatment, a step of salifying the compound as shown in Formula (I) is further comprised. In some embodiments, the salifying refers to reacting the compound as shown in Formula (I) with an acid reagent to obtain a salt of the compound as shown in Formula (I).

It has been found in studies that the selection of the acid reagent used has a great influence on the product's stability, salification ratio, filtration rate, and the removal efficiency of isomeric impurities. In some embodiments, the acid used includes hydrochloric acid, hydrobromic acid, methanesulfonic acid, p-toluenesulfonic acid, camphorsulfonic acid, phosphoric acid, acetic acid, benzoic acid, sulfuric acid and TFA, preferably methanesulfonic acid.

In some embodiments, the reaction solvent is one of water, methanol, ethanol, isopropanol, acetonitrile, THF and 1,4-dioxane or any combination thereof, preferably a mixed solvent system of methanol and water.

It has been found in studies that the order of reagent addition greatly influence reaction stirring and temperature control. When methanesulfonic acid or water is added last, the reaction solution releases heat intensely, making it difficult to control the temperature and stir. In the preferred experimental scheme, methanesulfonic acid is slowly added to a mixed solvent of water and MeOH, and finally a crude free-base product is added in batches. Throughout the process, stirring is effective, and the reaction temperature is easily manageable.

In some embodiments, the reaction temperature is 20 to 80 °C, preferably 40 to 55 °C. Under this temperature condition, stirring is effective, salification is complete, and the product is stable.

In some embodiments, after the salification, a post-treatment step is further comprised.

In some embodiments, the post-reaction step comprises filtration, such as direct filtration, filtration after adding and mixing with water, filtration after adding and mixing with methanol, filtration after adding and mixing with EtOH, filtration after adding and mixing with isopropanol, filtration after adding and mixing with acetone, or filtration after adding and mixing with acetonitrile, preferably adding methanol for crystallization and filtration.

In some embodiments, the method further comprises the step of preparing the compound as shown in Formula (IV) according to any of the methods described above.

In some embodiments, the method further comprises the step of preparing the compound as shown in Formula (III) according to any of the methods described above.

In another aspect, the present application provides a method for preparing a drug-linker, which comprises the method for preparing the compound as shown in Formula (I) or a salt thereof according to any of the methods described above, wherein the drug is the compound as shown in Formula (I) or a salt thereof.

In some embodiments, the method further comprises the step of preparing the compound as shown in Formula (IV) according to any of the methods described above.

In some embodiments, the method further comprises the step of preparing the compound as shown in Formula (III) according to any of the methods described above.

In another aspect, the present application provides a method for preparing an antibody-drug conjugate, comprising reacting the antibody with the drug-linker, in which the drug-linker is prepared according to the method described above.

### Beneficial effects of the present invention

The present application provides a method for preparing exatecan and its derivatives, by optimizing the types and equivalents of reaction reagents, reaction solvents and temperatures, developing isomer inversion conditions, avoiding high-temperature and long-duration reactions, the total yield of the three-step synthesis is increased from 43% to more than 75%, resulting in a savings of 1/3 of the material costs as compared to the original process. In addition, the substrate for configuration inversion has broad applicability and can be used for the synthesis of various exatecan compounds and their analogs.

### Brief Description of the Drawings

The drawings described herein are used to provide a further understanding of the present invention and constitute a part of the present application. The schematic examples of the present invention and their descriptions are used to explain the present invention and do not constitute an improper limitation of the present invention. In the drawings:

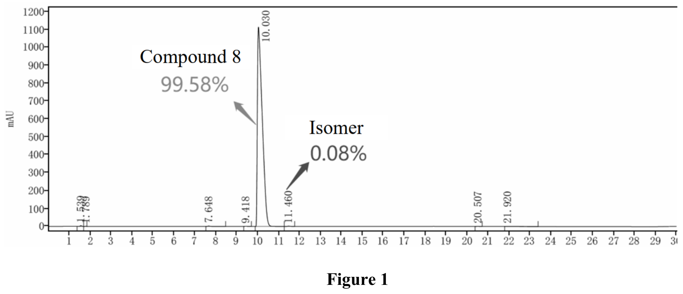

Figure 1 shows the HPLC diagram of Compound **8** prepared in Example 3.

### Specific Models for Carrying Out the present invention

The following will describe the embodiments of the present invention in detail with reference to the examples, but those skilled in the art will understand that the following examples are only used to illustrate the present invention and should not be considered as limiting the scope of the present invention. If specific conditions were not specified in the examples, the conventional conditions or the conditions recommended by the manufacturer should be followed. If the manufacturer of the reagents or instruments used was not specified, they were all conventional products that could be purchased commercially.

The abbreviations and English terms used herein have the following meanings:

| Abbreviation | Meaning | Abbreviation | Meaning |
|---|---|---|---|
| PPA | Polyphosphoric acid | MTBE | Methyl tert-butyl ether |
| 2-MeTHF | 2-Methyltetrahydrofuran | PPTS | Pyridinium p-toluenesulfonate |
| CAN | Cerium ammonium nitrate | T₃P | 1-Propylphosphonic anhydride |
| m-cresol | m-Cresol | o-cresol | o-Cresol |
| DPP | Diphenylphosphoric acid | Hep | n-Heptane |
| AcOH | Acetic acid | TFA | Trifluoroacetic acid |
| ND | Not detected | TsOH | p-Toluenesulfonic acid |
| EtOH | Ethanol | PhMe | Toluene |
| Proline | Proline | TMSC1 | Trimethylchlorosilane |
| BuOH | n-Butanol | iPrOAc | Isopropyl acetate |
| DMA | N,N-Dimethylacetamide | DMF | N,N-Dimethylformamide |
| NMP | N-Methylpyrrolidone | PhOMe | anisole |

### Example 1-1: Preparation of Compound 6

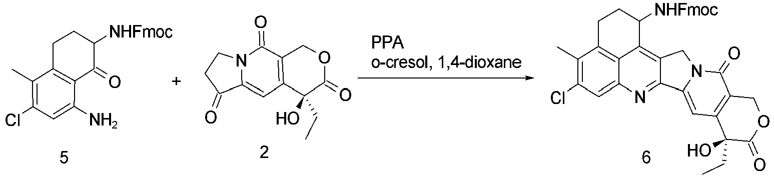

PPA (68.05 g, 201.38 mmol), o-cresol (360 mL) and 1,4-dioxane (360 mL) were added into a 2L glass reaction bottle in sequence, and stirring was started. Compound **5** (90.00 g, 201.38 mmol) and Compound **2** (58.31 mmol, 221.51 mmol) were added. After replacing the air with nitrogen gas three times, stirring was continued in N₂ environment, and heating was started. The internal temperature was maintained at 85 to 95 °C, and the stirring was continued for 12 to 18 hours. The reaction was stopped when HPLC detection showed that Compound **5** was less than 1.0%.

Post-treatment: The reaction solution was cooled to <60 °C, transferred, and then concentrated under reduced pressure at 50 to 60 °C until no more liquid was visibly dripped out, marking the completion of the concentration. The concentrate was transferred to a 10L glass bottle, stirring was started, and MTBE (5L) was slowly added to precipitate a large amount of solid, followed by a slow addition of n-heptane (1.4L). After the addition, stirring was continued for 1 to 2 hours. Filtration and drying by suction were performed. The filter cake was rinsed twice with MTBE (500mL*2) and subjected to suction filtration until no more liquid was visibly dripped out. The filter cake was collected and dried in vacuum at 40 to 50°C for 16 to 24 hours. The material was collected to obtain 152.66g of crude product of Compound **6** (yield: 112.5%, HPLC: 96.44% (46.62%+49.82%), qNMR: 88%).

Washing: 150g of the crude product of Compound **6** was taken, added with 2-MeTHF (4500mL), stirred at room temperature for dissolution, continuously added with NaCl aqueous solution (1500mL), stirred for 5 to 15 minutes, and allowed to stand for stratification; the organic phase was separated, added with 2% sodium sulfite aqueous solution (750mL) and saturated brine (750mL), stirred for 5 to 10 minutes, and allowed to stand for stratification; the organic phase was separated, added with 2% sodium carbonate aqueous solution (750mL) and saturated brine (750mL), stirred for 5 to 10 minutes, and allowed to stand for stratification; the organic phase was separated, added with water (750mL) and saturated brine (750mL), stirred for 5 to 10 minutes, and allowed to stand for stratification; the organic phase was separated, and concentrated under reduced pressure at 35 to 45 °C until no obvious liquid dripped out to obtain the washed Compound **6.**

Refining: The above-mentioned washed product was dissolved in 2-MeTHF (450mL), heated to 40 to 60 °C (gradually dissolved until clear, then some solids were precipitated out), stirred for 10 to 20 minutes; slowly added with MTBE (1800mL); after the addition, slowly cooled to 20 to 30 °C, the stirring was continued for 1 to 3 hours. Filtration and drying by suction were performed, the filter cake was rinsed once with MTBE (300mL), and subjected to suction filtration to dryness; the filter cake was collected, vacuum dried at 40 to 50 °C for 16 to 24 hours; the material was collected to obtain 120g of Compound **6** (total yield 89%, HPLC: 99.17% (48.75%, 50.42%), QNMR: 96%).

### Example 1-2: Preparation of Compound 6

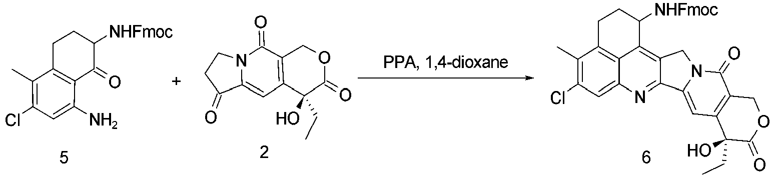

PPA (227mg, 1.5eq) and 1,4-dioxane (2ml) were added into a glass reaction bottle in sequence, and stirring was started. Compound **5** (200 mg) and Compound **2** (140 mg) were added. After replacing the air with nitrogen gas three times, the stirring was continued in N₂ environment, and heating was started. The internal temperature was maintained at 85 to 95 °C, and the stirring was continued for 12 to 18 hours. The reaction was stopped when HPLC detection showed that Compound **5** was less than 1.0%.

Post-treatment: The reaction solution was cooled to <60 °C, transferred, and then concentrated under reduced pressure at 50 to 60 °C until no more liquid was visibly dripped out, marking the completion of the concentration. 10 mL of MTBE and 3 mL of n-heptane were slowly added, and the stirring was continued for 1 to 2 hours. Filtration and drying by suction were performed. The filter cake was rinsed twice with MTBE and subjected to suction filtration until no more liquid was visibly dripped out. The filter cake was collected, and vacuum dried at 40 to 50 °C for 16 to 24 hours; the material was collected to obtain 370 mg of a crude product of Compound **6** (content about 75%).

### Example 2: Preparation of Compound 7

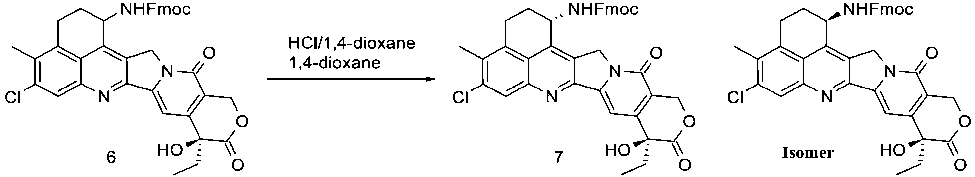

Compound **6** (114.00g, 169.10mmol) and 1,4-dioxane (2280mL) were added into a 5L glass bottle in sequence, and stirring was started; HCl/1,4-dioxane (274mL, 1096mmol, 4M) was added, and heating was started. The internal temperature was controlled at 40 to 50 °C, stirring was continued for 6 to 10 hours, and the reaction was stopped when HPLC detection showed that the ratio of Compound **6:** isomer was 10:1 to 25:1.

Post-treatment: The reaction solution was cooled to 20 to 30 °C, added with MTBE (2600mL) slowly, cooled to 15 to 20 °C after the addition, and stirring was continued for 1 to 3 hours. Filtration and drying by suction were performed; the filter case was rinsed with MTBE (330mL*3) three times and subjected to suction filtration to dryness. The filter cake was collected, and vacuum dried at 40 to 50 °C for 16 to 24 hours. The material was collected to obtain 108.3g of product (yield: 95%, HPLC: 97.18% Compound 7 and 2.12% isomers, QNMR: 99%).

### Example 3: Preparation of Compound 8

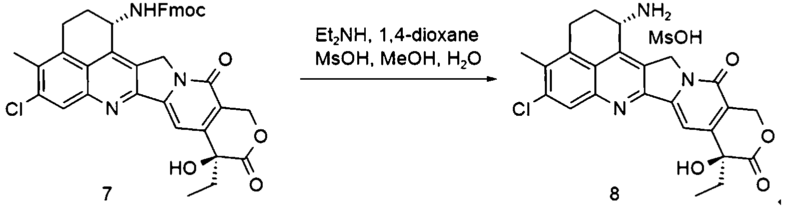

Compound **7** (104.44g, 154.92mmol) and 1,4-dioxane (627mL) were added into a 1L glass bottle in sequence, and stirring was started. Et₂NH (56.65g, 774.62mmol) was added slowly while controlling the internal temperature at 20 to 30 °C. After the addition, stirring was continued at this temperature for 18 to 24 hours. The reaction was stopped when HPLC detection showed that the content of Compound 7 was less than 1%,.

Post-treatment: The reaction solution was transferred, concentrated under reduced pressure at 40 to 50 °C to about 1/3 of the volume of the reaction solution, acetonitrile (530mL) was added, and slurry washing was conducted at room temperature for 1 to 2 hours, and then the reduced pressure concentration was continued until no more liquid was visibly dripped out, marking the completion of the concentration. Acetonitrile (1060 mL) was added and heated to 65 to 75 °C, and slurry washing was conducted for 2 hours; after slowly cooling to 20 to 30 °C, the stirring was continued for 8 to 16 hours. Filtration and drying by suction were performed, the filter cake was rinsed with acetonitrile (200 mL) once. The filter cake was collected and added with acetonitrile (1060mL), slurry washing was conducted at 65 to 75 °C for 2 hours; after slowly cooling to 20 to 30 °C, the stirring was continued for 3 to 5 hours. filtration and drying by suction were performed, the filter cake was rinsed once with acetonitrile (200mL). The filter cake was collected and dried in vacuum at 40 to 50 °C for 16 to 24 hours; the material was collected to obtain 67g of a crude product of the free alkali.

Salification: Water (255mL) and methanol (126mL) were added to a 3L glass bottle, and stirring was started; methanesulfonic acid (255mL) was slowly added, and the internal temperature was controlled at 20 to 30 °C; after addition, the above crude product of the free alkali (60g, 132.77mmol) was added in three batches, heated to 40 to 50 °C for reaction, and stirred for 1 to 2.5 hours. A mixed solution of methanesulfonic acid (126mL) and methanol (126mL) were slowly added, and the stirring was continued for 1 to 2 hours. Methanol (1070mL) was slowly added to precipitate a large amount of solid; after addition, the stirring was continued for 1 to 2 hours; cooling was performed to 20 to 30 °C, and stirring was continued for 3 to 5 hours.

Filtration and drying by suction, the filter cake was rinsed once with methanol (300mL) and subjected to suction filtration; the filter cake was collected and dried in vacuum at 40 to 50 °C for 18 to 24 hours. The material was collected to obtain 65.12g product (yield: 90%, HPLC: 99.58% Compound **8** and 0.08% isomer, QNMR: 98%).

HPLC of Compound **8** was shown in Figure 1.

### Example 4: Reagents screening experiment for the cyclization reaction of Step 1

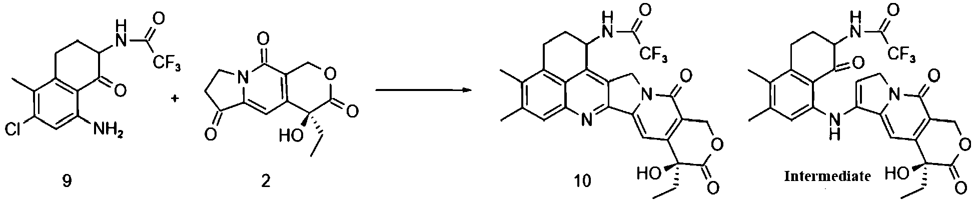

Compound **9** was used as the model substrate, 100mg of which was charged for each reaction, and reacted with Compound **2** in 1ml of solvent to prepare the target Compound **10,** progress of the reaction was monitored by HPLC, and the results after 4 hours of reaction were shown in Table 1.

**Table 1. Results of cyclization reagent screening**

| No. | Reaction condition | | Temperature (°C) | Compound **9** (%) | Compound **2** (%) | Product **10** (%) | Intermediat e (%) |
|---|---|---|---|---|---|---|---|
| 1 | AcOH | | 90 | 69.955 | 17.987 | 2.42 (1:0.84)* | 2.126 |
| 2 | TFA | | 80 | 84.162 | ND | ND | ND |
| 3 | o-cresol | | 90 | 77.906 | 19.028 | ND | ND |
| 4 | m-cresol | | 90 | 79.576 | 19.429 | 0.066 | ND |
| 5 | H₂SO₄ (0.5eq), AcOH | | 90 | 44.510 | 11.925 | 30.82 (1:0.99) | 0.994 |
| 6 | TsOH·H₂O(0.5eq), AcOH | | 90 | 39.404 | 10.470 | 25.51 (1:0.84) | 7.305 |
| 7 | TsOH·H₂O (0.5eq) | EtOH | 80 | 26.152 | 3.159 | 1.697 | 39.082 |
| 8 | | m-cresol | 90 | 59.902 | 15.375 | 20.33 (1:1.12) | 1.006 |
| 9 | | PhMe | 90 | 59.663 | 14.716 | 0.103 | 19.296 |
| 10 | | AcOH/P hMe | 90 | 28.818 | 28.054 | 25.91 (1:0.93) | 5.396 |
| 11 | MsOH (0.5eq), PhMe | | 90 | 64.929 | 16.636 | 0.081 | 15.296 |
| 12 | PPTS (0.5eq), PhMe | | 90 | 20.592 | 7.999 | 0.481 | 37.141 |
| 13 | CAN (0.5eq), EtOH | | 80 | 30.918 | 2.346 | 16.50 (1:0.72) | 14.489 |
| 14 | T₃P (0.5eq), DMF | | 90 | 56.355 | 16.084 | 2.94 (1:0.21) | 0.552 |
| 15 | proline (0.5eq), DMSO | | 90 | 93.399 | 3.623 | ND | ND |
| 16 | KOH (0.5eq), EtOH | | 80 | 95.931 | 0.871 | 0.097 | 0.067 |
| 17 | I₂ (0.5eq), DMF | | 80 | 32.753 | 8.014 | 0.533 | 0.659 |
| 18 | MgCl₂, (0.5eq), TsOH·H₂O, ACN | | 80 | 55.247 | 18.239 | 4.23 (1:1.78) | 5.639 |
| 19 | TMSCl (3eq), DMF | | 100 | 41.482 | 4.115 | 34.22 (1:0.75) | 0.042 |
| 21 | PPA(0.5eq), m-cresol | | 90 | 32.564 | 8.707 | 41.58 (1:1.34) | 12.404 |
| 22 | PPTS, o-cresol, PhMe | | 110 | 28.28 | 4.20 | 25.25 (1:1.54) | 14.33 |
| 23 | PPA(0.5eq), o-cresol | | 90 | 8.56 | 2.42 | 49.45 (1:1.11) | 3.18 |
| 24 | PPA(0.3eq), AcOH/PhMe | | 90 | 31.683 | 10.548 | 23.92 (1:0.88) | 9.624 |
| 25 | H₂PO₄(85%), o-cresol | | 90 | 28.13 | 7.81 | 15.80 (1:1.14) | 3.96 |
| 26 | DPP, o-cresol | | 90 | 30.21 | 6.71 | 16.20 (1:0.97) | 1.54 |
| 27 | TsOH(0.5eq), Ac₂O/AcOH/PhMe | | 90 | The reaction was very complicated. | | | |

| | | | | | | | |
|---|---|---|---|---|---|---|---|
| Note: *The ratio of diastereomers of Product 10, i.e., (1S, 9S)-product **10** to (1R, 9S)-product **10.** | | | | | | | |

The above table data showed that reaction **23** (PPA, o-crestol) was the fastest reaction, had fewer impurities, and had a better price advantage than the combination of PPA and m-crestol (reaction **21**), and can be further optimized as a better condition.

### Example 5: Solvents screening experiment for the cyclization of Step 1

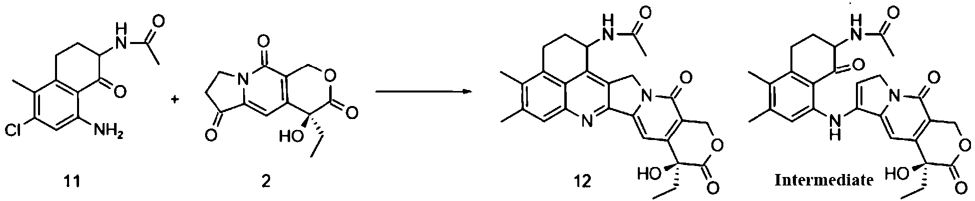

Compound **11** was used as the model substrate, 100 mg of which was charged for each reaction, 1 equivalent of PPA was added and reacted with Compound **2** to prepare the target compound **12,** progress of the reaction was monitored by HPLC, and the results of the reaction at 95 °C for 11 hours were shown in Table 2.

**Table 2. Results of solvent screening for first step of cyclization reaction**

| No. | Reaction condition | Compound **11** (%) | Compound **2** (%) | Compound **12** (%) | Intermediate (%) |
|---|---|---|---|---|---|
| 1 | 1mL o-cresol | 6.23 | 2.50 | 50.42 (0.81:1)* | 0.63 |
| 2 | 0.5mL o-cresol | 4.47 | 1.32 | 69.40 (0.99:1) | ND |
| 3 | 0.5mL o-cresol, 0.5mL BuOH | 8.95 | 1.55 | 63.34 (0.67:1) | 0.61 |
| 4 | 0.5mL o-cresol, 0.5mL iPrOAc | 9.96 | 5.67 | 51.01 (0.84:1) | 1.97 |
| 5 | 0.5mL o-cresol, 0.5mL 1,4-dioxane | 6.67 | 1.76 | 68.28 (0.84:1) | ND |
| 6 | 0.5mL o-cresol, 0.5mL Hep | 5.26 | 4.43 | 56.08 (0.72:1) | 6.89 |
| 7 | 0.5mL o-cresol, 0.5mL DMA | 16.37 | 2.32 | 53.10 (0.99:1) | ND |
| 8 | 0.5mL o-cresol, 0.5mL DMF | 27.70 | 4.34 | 29.10 (0.95:1) | ND |
| 9 | 0.5mL o-cresol, 0.5mL DMSO | 12.55 | 2.45 | 54.28 (0.99:1) | ND |
| 10 | 0.5mL o-cresol, 0.5mL NMP | 11.49 | 1.50 | 61.10 (0.99:1) | ND |
| 11 | 0.5mL o-cresol, 0.5mL PhOMe | 8.66 | 3.52 | 31.83 (0.84:1) | 2.97 |
| 12 | 0.3mL o-cresol, 0.7mL 1,4-dioxane | 7.31 | 1.65 | 31.21 (0.85:1) | ND |
| 14 | 1.2mL o-cresol, 0.3mL 1,4-dioxane | 6.61 | 1.74 | 68.28 (0.84:1) | ND |

| | | | | | |
|---|---|---|---|---|---|
| Note: *The ratio of diastereomers of Product **12,** i.e., (1S, 9S)-product **12** to (1R, 9S)-product **12.** | | | | | |

The above table data showed that as compared with single solvent, the mixed solvent system of o-cresol and 1,4-dioxane had the best reaction.

### Example 6: Conditional screening experiment for inversion of configuration of Step 2

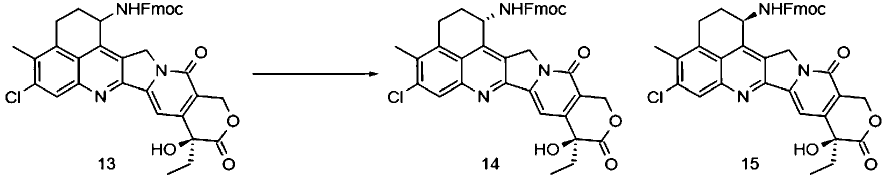

Compound **13** was used as the model substrate, 50 mg of which was charged for each reaction, and 1 ml of solvent was used to screen the ratio changes of products **14** to **15** under different conditions. The results were as follows:

| No. | Reaction condition | Temperature/°C | Time/ h | Compound 14/Compound 15 |
|---|---|---|---|---|
| 1 | 6N Hydrochloride acid aqueous solution | 70 | 2 | 0.99 |
| | | | 17 | 0.98 |
| | | | 25 | 0.87 |
| | | | 40 | 0.37 |
| 2 | AcOH | 70 | 2 | 0.62 |
| | | | 17 | 0.64 |
| | | | 25 | 0.70 |
| | | | 40 | 0.70 |
| 3 | TFA | 70 | 2 | 0.75 |
| | | | 17 | 0.80 |
| | | | 25 | 0.81 |
| | | | 40 | 0.94 |
| 4 | MsOH | 70 | 2 | 0.68 |
| | | | 17 | 0.66 |
| | | | 25 | 0.65 |
| | | | 40 | 0.50 |
| 5 | H₃PO₄ | 70 | 2 | 0.69 |
| | | | 17 | 0.80 |
| | | | 25 | 0.83 |
| | | | 40 | 0.87 |
| 6 | H₂SO₄ | 70 | 2 | 0.96 |
| | | | 17 | 0.92 |
| | | | 25 | 0.90 |
| | | | 40 | 0.84 |
| 7 | HCl/dioxane (4M) | 70 | 2 | 2.81 |
| | | | 17 | 2.36 |
| | | | 25 | 1.00 |
| | | | 40 | 0.78 |
| 8 | HCl/dioxane (4M) | 45 | 0 | 0.72 |
| | | | 1 | 5.36 |
| | | | 2.5 | 6.49 |
| | | | 17.5 | 14.40 |

The above tabular data showed that, under the conditions of HCl/dioxane, at the reaction temperature of 45°C, the target Compound **14** had the highest ratio.

Although the specific embodiments of the present invention had been described in detail, those skilled in the art will understand that various modifications and substitutions can be made to those details based on all the teachings disclosed, and these changes are within the scope of protection of the present invention. The full scope of the present invention is given by the appended claims and any equivalents thereof.

## Claims

1. A method for preparing a compound as shown in Formula (III), comprising a step of reacting a compound as shown in Formula (II) and Compound 2 in the presence of a catalyst; 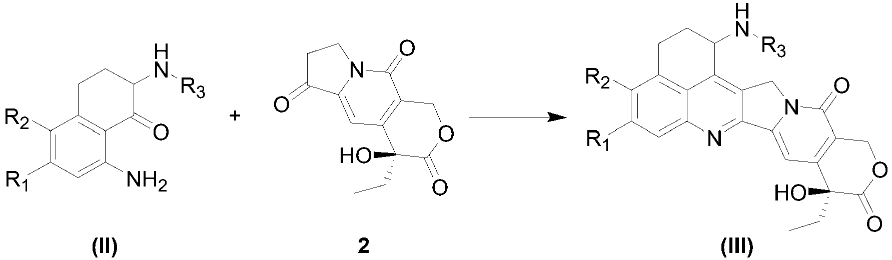
wherein, R₁ and R₂ are independently selected from the group consisting of hydrogen, halogen, C₁₋₆ alkyl, C₁₋₆ hydroxyalkyl, C₁₋₆ alkoxyalkyl, C₁₋₆ alkoxy, C₁₋₄ haloalkyl, hydroxyl and cyano; or R₁ and R₂, together with the carbon atoms to which they are connected, form a 5-to 6-membered carbon ring or an oxygen-containing heterocyclic ring;
\R₃ is an electron-withdrawing protective group;
the catalyst is selected from one or more of PPTS, AcOH, TFA, H₂SO₄, proline, PPA, P₂O₅, CAN, T₃P, KOH, I₂, MgCl₂ and TMSCl.

2. The method according to claim 1, wherein the catalyst is PPA.

3. The method according to claim 1, wherein the compound as shown in Formula (II) and Compound **2** are reacted in a solvent, wherein the solvent is selected from the group consisting of aromatic hydrocarbons (e.g., toluene, xylene, o-toluene, m-toluene), aliphatic hydrocarbons (e.g., hexane, n-heptane), alcohols (e.g., methanol, ethanol, isopropanol), organic acids (e.g., acetic acid, trifluoroacetic acid), phenols (e.g., phenol, o-cresol, m-cresol, p-cresol), ethers (e.g., ethyl ether, ethylene oxide, anisole), esters (e.g., methyl acetate, ethyl acetate, propyl acetate), ketones (e.g., acetone, butanone), amides (e.g., DMF, DMA), nitriles (e.g., acetonitrile), heterocyclics (e.g., NMP, 1,4-dioxane, 2-MeTHF), sulfur-containing organic solvents (e.g., DMSO) and any combination thereof.

4. The method according to claim 3, wherein the solvent is a combination of a phenolic solvent and a heterocyclic solvent.

5. The method according to claim 4, wherein the solvent is a mixed solvent of o-cresol and 1,4-dioxane.

6. The method according to any one of claims 1 to 5, **characterized by** one or more of the following items:
(1-1) the volume ratio of o-cresol to 1,4-dioxane is (10:1) to (1:10);
(1-2) the feed molar ratio of the compound as shown in Formula (II) to Compound **2** is (1:1) to (1:1.5);
(1-3) based on the compound as shown in Formula (II), the feed equivalent of PPA is 0.2 to 5 equivalents;
(1-4) the compound as shown in Formula (II) and Compound **2** are reacted at 60 °C to 140 °C;
(1-5) the compound as shown in Formula (II) and Compound **2** are reacted for 6 to 48 hours;
(1-6) the reaction is carried out without gas protection, or with nitrogen protection and argon protection;
(1-7) after the reaction is completed, a step of post-treatment to obtain a crude product of the compound as shown in Formula (III) is further comprised;
(1-8) after obtaining the crude product of the compound as shown in Formula (III) described in item (1-7), a step of washing the crude product of the compound as shown in Formula (III) is further comprised;
(1-9) after performing the washing step described in item (1-8), a step of recrystallization is further comprised.

7. The method according to claim 6, wherein
the volume ratio of o-cresol to 1,4-dioxane is (10:1) to (1:1); and/or
the feed molar ratio of the compound as shown in Formula (II) to Compound **2** is (0.8:1) to (1:1.5).

8. The method according to claim 6, **characterized by** one or more of the following items:
1-1) the post-treatment step described in item (1-7) comprises adding water, sodium carbonate aqueous solution or sodium bicarbonate aqueous solution for quenching, and then filtering; or adding a mixed solvent of DCM and DCM-IPA or 2-MeTHF for extraction; or directly concentrating the reaction solution under reduced pressure to remove 1,4-dioxane, and then adding one or more solvents selected from isopropanol, ethyl acetate, acetone, MTBE, PhMe, n-heptane and ACN simultaneously or separately for dilution, and then filtering;
1-2) the crude product of the compound as shown in Formula (III) obtained in item (1-7) does not need to be dried;
1-3) the washing step described in item (1-8) comprises dissolving the crude product of the compound as shown in Formula (III) with an organic solvent to obtain an organic phase, washing the organic phase with an aqueous phase for 1 to 5 times, and concentrating the organic phase;
1-4) the recrystallization step described in item (1-9) comprises dissolving the compound as shown in Formula (III) with 2-MeTHF and precipitating crystals with MTBE.

9. A method for preparing a compound as shown in Formula (IV), comprising the step of converting a compound as shown in Formula (III) into a compound as shown in Formula (IV); 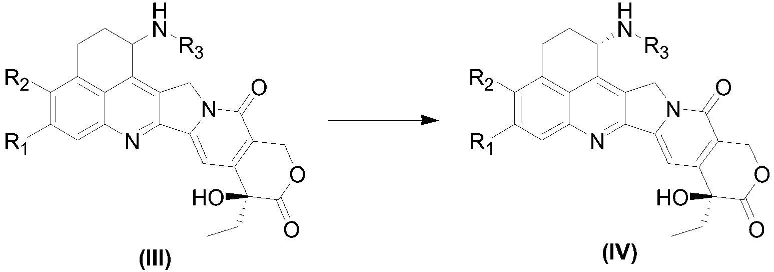 wherein R1, R₂ and R₃ are as defined in claim 1.

10. The method according to claim 9, wherein the conversion is carried out in an organic solvent selected from the group consisting of alcohols (e.g., methanol, ethanol, isopropanol, n-butanol), ketones (e.g., acetone, butanone, methyl isobutyl ketone), esters (e.g., ethyl acetate, isopropyl acetate), ethers (e.g., MTBE, isopropyl ether, anisole), aliphatic hydrocarbons (e.g., n-heptane), heterocyclics (e.g., THF, 1,4-dioxane, 2-MeTHF, NMP), amides (e.g., DMF, DMA, sulfur-containing organic solvents (e.g., DMSO), halogenated aliphatic hydrocarbons (e.g., DCM), ammoniums (e.g., CAN), aromatic hydrocarbons (e.g., toluene), and any combination thereof.

11. The method according to claim 9 or 10, wherein the conversion is carried out in the presence of an acid.

12. The method according to claim 11, wherein the acid is HCl.

13. The method according to any one of claims 9 to 12, **characterized by** one or more of the following items:
(2-1) the conversion is carried out in HCl/1,4-dioxane;
(2-2) based on the compound as shown in Formula (III), the acid feed is 2 to 20 equivalents;
(2-3) the conversion is carried out at 25 to 100 °C;
(2-4) the reaction is stopped when the ratio of the compound as shown in Formula (IV) to its isomer is (2:1) to (30:1);
(2-5) the conversion is carried out for 1 to 24 hours;
(2-6) after the conversion, the method further comprises a post-treatment step;
(2-7) the method further comprises a step of preparing the compound as shown in Formula (III) according to the method according to any one of claims 1 to 8.

14. The method according to claim 13, **characterized by** one or more of the following items:
2-1) the post-treatment step described in item (2-7) involves directly concentrating under reduced pressure, or adding one or more anti-solvents of the organic solvent described in claim 10 to precipitate more solids, and then filtering to obtain the target product;
2-2) the anti-solvent described in item 2-1) is one or more of ethanol, isopropanol, n-butanol, acetone, butanone, methyl isobutyl ketone, ethyl acetate, isopropyl acetate, MTBE, n-heptane, THF, 1,4-dioxane, isopropyl ether, anisole, DCM, ACN and toluene.

15. A method for preparing a compound as shown in Formula (I) or a salt thereof, comprising a step of removing the amino protective group of a compound as shown in Formula (IV) to obtain a compound as shown in Formula (I); 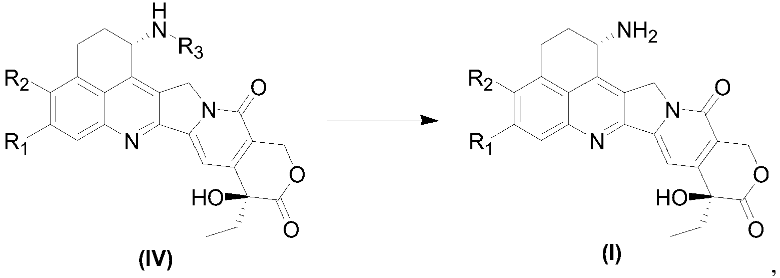 wherein, R₁, R₂ and R₃ are as defined in claim 1.

16. The method according to claim 15, **characterized by** one or more of the following items:
(3-1) after removing the amino protective group of the compound as shown in Formula (IV), the method further comprises a post-treatment step;
(3-2) after the post-treatment described in item (3-1), the method further comprises a step of salifying the compound as shown in Formula (I);
(3-3) after the salification described in item (3-2), the method further comprises a post-treatment step;
(3-4) the method further comprises a step of preparing the compound as shown in Formula (IV) by the method according to any one of claims 9 to 14;
(3-5) the method further comprises a step of preparing the compound as shown in Formula (III) by the method according to any one of claims 1 to 8.

17. The method according to claim 16, **characterized by** one or more of the following items:
3-1) the post-treatment step described in item (3-1) involves concentrating, slurry washing and filtering 1 to 3 times to obtain a crude product of the compound as shown in Formula (I);
3-2) the concentration step described in item 3-1) refers to concentrating to 1/4-1/2 of the volume of the original solution, followed by mixing with acetonitrile to form a slurry, and further concentrating until dryness;
3-3) the slurry washing described in item 3-1) is carried out at a high temperature of 60 to 80 °C and a low temperature of 10 to 40 °C in sequence, and then the solid is collected by filtration to obtain the crude product of the compound as shown in Formula (I);
3-4) the salification described in item (3-2) comprises reacting the compound as shown in Formula (I) with an acid reagent to obtain a salt of the compound as shown in Formula (I);
3-5) the post-treatment step described in item (3-3) is filtration, such as direct filtration, filtration after adding and mixing with water, filtration after adding and mixing with methanol, filtration after adding and mixing with EtOH, filtration after adding and mixing with isopropanol, filtration after adding and mixing with acetone or filtration after adding and mixing with acetonitrile.

18. A method for preparing a drug-linker, comprising the step of preparing the compound as shown in Formula (I) or a salt thereof according to any one of claims 15 to 17, wherein the drug is the compound as shown in Formula (I) or a salt thereof.

19. A method for preparing an antibody-drug conjugate, which is prepared by reacting the antibody and the drug-linker, and further comprises the step of preparing the drug-linker according to the method of claim 18.
